# EUROPEAN PATENT APPLICATION

(11) **EP 4 803 614 A1**
(43) Date of publication of application: **09.09.2026**
(21) Application number: 24884692.5
(22) Date of filing: 29.10.2024
(51) Int. Cl.: C12N 5/10, C07K 19/00, A61P 35/00

(54) **CELL EXPRESSING MULTISPECIFIC ANTIGEN BINDING PROTEIN AND USE THEREOF**

(30) Priority: 30.10.2023 WO PCT/CN2023/127553
(71) Applicant: Beijing Grit Biotherapeutics Co., Ltd., Shanghai 200120 (CN); Shanghai Grit Biotechnology Co., Ltd., Shanghai 200120 (CN); Suzhou Grit Biotechnology Co., Ltd., Shanghai 200120 (CN)
(72) Inventor: LIU, Yarong, Shanghai 200120 (CN); SUN, Jingwei, Shanghai 200120 (CN); TAN, Yiyang, Shanghai 200120 (CN)
(74) Representative: Fish & Richardson P.C.
(86) International application number: PCT/CN2024/127934
(87) International publication number: WO 2025/092689

(57) **Abstract**

The present invention relates to the field of biomedicine, and in particular relates to a cell expressing a multispecific antigen-binding protein and the use thereof. The cell has enhanced capabilities, such as cell proliferation capability, cytokine secretion capability, tumor cell killing capability, and the like.

## Description

### TECHNICAL FIELD

The present invention relates to the field of biomedicine, and in particular relates to a cell expressing a multispecific antigen-binding protein and an application thereof.

### BACKGROUND

Currently, immunotherapy is an effective method for treating patients with poor prognosis. However, immune cells used in immunotherapy have problems in that, after being reinfused in vivo, cell function is not strong, or proliferation and persistence capabilities are weak. Therefore, a modified immune cell and a robust and reliable culture method for immune cells are urgently needed to be provided.

Therefore, there is an urgent need in the art for a genetically engineered cell for successfully achieving genetic engineering and for improving the function of the cell through genetic engineering.

### SUMMARY

The present invention provides an isolated cell comprising a multispecific antigen-binding protein expressed therein. The cell expressing the multispecific antigen-binding protein according to the present invention has one or more of the following characteristics: (1) improved cell proliferative capacity; (2) enhanced cytokine secretion capacity; (3) improved tumor cell killing capacity; (4) improved or stable exogenous gene transduction efficiency; (5) improved downstream signaling pathway strength; and/or (6) improved cell subpopulation proportions, for example, an increased proportion of activated cells, a reduced proportion of regulatory cells, a reduced proportion of exhausted cells, an increased proportion of central memory cells and/or naïve cells, a reduced proportion of apoptotic cells, and an increased proportion of stem cell-like cells.

In another aspect, the present invention provides a method for producing the cell of the present invention, comprising causing the cell to artificially express the multispecific antigen-binding protein.

In another aspect, the present invention provides a composition comprising the cell of the present invention, and optionally a pharmaceutically acceptable carrier.

In another aspect, the present invention provides a kit, wherein the kit comprises the cell of the present invention and/or the composition of the present invention.

In another aspect, the present invention provides use of the cell of the present invention, the composition of the present invention, and/or the kit of the present invention in the manufacture of a medicament, wherein the medicament is used for preventing, alleviating, and/or treating a disease, preferably a tumor.

In another aspect, the present invention provides a medicament for preventing and/or treating a disease and/or symptom, preferably a tumor, comprising the cell of the present invention, the composition of the present invention, and/or the kit of the present invention.

In another aspect, the present invention provides a method for preventing and/or treating a disease and/or symptom, preferably a tumor, comprising administering to a subject in need thereof the cell of the present invention, the composition of the present invention, and/or the kit of the present invention.

In another aspect, the present invention provides the cell of the present invention, the composition of the present invention, and/or the kit of the present invention for use in preventing and/or treating a disease and/or symptom, preferably a tumor.

Other aspects and advantages of the present invention are readily apparent to those skilled in the art from the following detailed description. Only exemplary embodiments of the present invention are shown and described in the following detailed description. As will be recognized by those skilled in the art, the disclosure of the present invention enables those skilled in the art to make modifications to the disclosed specific embodiments without departing from the spirit and scope of the present invention. Accordingly, the drawings and the description in the specification of the present invention are merely exemplary and are not limiting.

### BRIEF DESCRIPTION OF THE DRAWINGS

Specific features of the present invention are shown in the appended claims. The features and advantages of the present invention are better understood with reference to the exemplary embodiments described in detail below and the accompanying drawings. The drawings are briefly described as follows:
**FIGs. 1A-1B** show transduction results of TIL cells from different donor sources.
**FIGs. 2A-2D** show results of cytotoxicity assays of TIL cells transduced with the target polypeptide of the present invention.
**FIGs. 3A-3E** show concentrations of various cytokines in supernatants after each group of TIL cells is co-incubated with A375 or Caski cells for 24 hours.
**FIGs. 4A-4D** show concentrations of various cytokines in supernatants after each group of TIL cells is co-incubated with A375 cells for 24 hours.

### DETAILED DESCRIPTION

The embodiments of the present invention are described below by specific embodiments, and other advantages and effects of the present invention may be readily understood by persons skilled in the art from the content disclosed in this specification.

### Definition of Terms

In the present invention, the term "EGFR" generally comprises epidermal growth factor receptor. For example, the UniProt number of EGFR may be P00533. The EGFR of the present invention may further encompass functionally active fragments thereof, without limitation to human and mammalian forms, conservative amino acid substitutions, glycoform modifications or variants, active fragments thereof, or variants comprising the functionally active fragments produced after processing and/or modification occurring in cells.

In the present invention, the term "BCMA" generally comprises B-cell maturation antigen. For example, "BCMA" may be used interchangeably with TNFRSF17, and the UniProt accession number for BCMA may be Q02223. The BCMA of the present invention may also encompass functionally active fragments thereof, including, without limitation, human and mammalian forms, conservative amino acid substitutions, glycoform modifications or variants, active fragments thereof, or variants comprising such functionally active fragments that are generated through processing and/or post-translational modification in cells.

In the present invention, the term "DLL3" generally comprises Delta-like protein 3. For example, the UniProt number of DLL3 may be Q9NYJ7. The DLL3 of the present invention may further encompass functionally active fragments thereof, without limitation to human and mammalian forms, conservative amino acid substitutions, glycoform modifications or variants, active fragments thereof, or variants comprising the functionally active fragments produced after processing and/or modification occurring in cells.

In the present invention, the term "FAP" generally comprises fibroblast activation protein. For example, the UniProt number of FAP may be Q12884. The FAP of the present invention may further encompass functionally active fragments thereof, without limitation to human and mammalian forms, conservative amino acid substitutions, glycoform modifications or variants, active fragments thereof, or variants comprising the functionally active fragments produced after processing and/or modification occurring in cells.

In the present invention, the term "peptide linker" may generally be used interchangeably with the term "linker". For example, a peptide linker is a peptide that covalently or non-covalently links two or more molecules or peptides, thereby producing a larger complex composed of all molecules or peptides including the peptide linker.

In the present invention, the term "fusion protein" generally refers to a protein composed of two or more molecules or peptides, which are generally not associated in a native state, but whose respective N-termini and C-termini may be directly or indirectly linked together to form a continuous polypeptide. For example, a portion of the fusion protein may be referred to as a "first polypeptide". For example, another portion of the fusion protein may be referred to as a "second polypeptide". For example, the fusion protein may comprise two or more portions, and the "first polypeptide" or "second polypeptide" does not limit the number of components of the fusion protein.

In the present invention, the term "antigen-binding protein" is used in its broadest sense and means a protein comprising a portion that binds to an antigen or target and optionally comprising a scaffold or framework portion that allows the antigen-binding portion to adopt a conformation that promotes binding of the antigen-binding protein to the antigen. Examples of antigen-binding proteins include human antibodies, humanized antibodies, chimeric antibodies, recombinant antibodies, single-chain antibodies, bifunctional antibodies, trifunctional antibodies, quadrifunctional antibodies, Fab fragments, F(ab')₂ fragments, IgD antibodies, IgE antibodies, IgM antibodies, IgG1 antibodies, IgG2 antibodies, IgG3 antibodies, or IgG4 antibodies, and fragments thereof. The antigen-binding protein may include, for example, alternative protein scaffolds or artificial scaffolds having grafted CDRs or CDR derivatives. Such scaffolds include, but are not limited to: antibody-derived scaffolds comprising mutations introduced, for example, to stabilize the three-dimensional structure of the antigen-binding protein; and fully synthetic scaffolds comprising, for example, biocompatible polymers.

In the present invention, the term "antibody" is used in its broadest sense, and specifically encompasses, but is not limited to, monoclonal antibodies (including full-length monoclonal antibodies comprising two light chains and two heavy chains), polyclonal antibodies, multispecific antibodies (e.g., bispecific antibodies), humanized antibodies, fully human antibodies, chimeric antibodies, heavy-chain antibodies, and camelized single-domain antibodies (e.g., heavy-chain variable domain antibodies).

In the present invention, the term "variable" generally refers to the fact that certain portions of the sequence of the variable domains of antibodies vary greatly, thereby forming the binding and specificity of various particular antibodies for their particular antigens. However, variability is not evenly distributed throughout the entire variable region of an antibody. It is concentrated in three segments in the light-chain and heavy-chain variable regions, which are referred to as complementarity determining regions (CDRs) or hypervariable regions (HVR). The more highly conserved portions of the variable domains are referred to as framework (FR). The variable domains of native heavy and light chains each comprise four FR regions, most of which adopt a β-pleated sheet configuration, are connected by three CDRs to form loop connections, and in some cases form part of the β-pleated sheet structure. The CDRs in each chain are brought into close proximity by the FR regions and, together with the CDRs from the other chain, form the antigen-binding site of the antibody. The constant regions do not directly participate in antibody-antigen binding, but exhibit different effector functions, such as participating in antibody-dependent cellular cytotoxicity. In the art, the CDRs of an antibody may be defined by various methods, for example, the Kabat definition rules based on sequence variability.

In the present invention, the term "Fab" refers to an antigen-binding fragment of an antibody. As described above, an intact antibody may be digested using papain. After papain digestion, the antibody produces two identical antigen-binding fragments, i.e., "Fab" fragments, and a residual "Fc" fragment (i.e., the Fc region, as above). A Fab fragment is composed of one complete L chain and the variable region of one heavy chain and the first constant region (C_{H}1) of the H chain (V_{H}).

In the present invention, the term "Fab' fragment" refers to a monovalent antigen-binding fragment of a human monoclonal antibody, which is slightly larger than a Fab fragment. For example, a Fab' fragment comprises all light chains, all heavy-chain variable regions, and all or part of the first and second constant regions of the heavy chain. For example, a Fab' fragment may further comprise part or all of 220-330 amino acid residues of the heavy chain.

In the present invention, the term "F(ab')₂" refers to an antibody fragment produced by pepsin digestion of an intact antibody. A F(ab')₂ fragment contains two Fab fragments held together by disulfide bonds and a portion of the hinge region. A F(ab')₂ fragment has bivalent antigen-binding activity and is capable of crosslinking antigens.

In the present invention, the term "Fv fragment" refers to a monovalent antigen-binding fragment of a human monoclonal antibody, comprising all or part of a heavy-chain variable region and a light-chain variable region, and lacking a heavy-chain constant region and a light-chain constant region. The heavy-chain variable region and the light-chain variable region comprise, for example, CDRs. For example, an Fv fragment comprises all or part of the approximately 110 amino acid N-terminal variable regions of the heavy and light chains.

In the present invention, the term "scFv" generally refers to a fusion protein comprising at least one antibody fragment comprising a light-chain variable region and at least one antibody fragment comprising a heavy-chain variable region, wherein the light-chain and heavy-chain variable regions are adjacent (e.g., via a synthetic linker such as a short flexible polypeptide linker), are capable of being expressed in the form of a single-chain polypeptide, and wherein the scFv retains the specificity of the intact antibody from which it is derived. Unless otherwise specified, the scFv may have the VL and VH variable regions in any order (e.g., relative to the N-terminus and C-terminus of the polypeptide), and the scFv may comprise VL-linker-VH or may comprise VH-linker-VL.

In the present invention, the amino acid residues may be naturally occurring amino acids and/or artificially modified amino acids. For example, naturally occurring amino acids may be selected from the group consisting of alanine (three-letter code: ala, one-letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

In the present invention, the term "immune cell" generally refers to a cell participating in an immune response, for example, promoting an immune effector response. Examples of immune cells include, but are not limited to, T cells, B cells, natural killer (NK) cells, NKT cells, mast cells, granulocytes, monocytes, lymphocytes, and macrophages. For example, the immune cells of the present invention may comprise cells derived from induced pluripotent stem (iPS) cells, PBMC cells, and/or tumor-infiltrating lymphocytes. For example, the immune cells of the present invention may be obtained by differentiation from iPS cells. The term further includes engineered immune cells, such as immune cells genetically modified by adding exogenous genetic material in the form of DNA or RNA to the total genetic material of the cells.

In the present invention, the term "killing capability" refers to killing cells by bringing the cells into contact with an effective amount of an antibody, T cells, an immunoconjugate, a bispecific/multispecific molecule, or a composition. For example, the fusion polypeptide of the present invention may enhance the killing capability of cells. For example, cells expressing the fusion polypeptide of the present invention may exhibit enhanced killing capability. For example, a combination of cells expressing the fusion polypeptide of the present invention with other immune cells, antibodies, immunoconjugates (e.g., conjugates of binding molecules targeting immune checkpoints with other active molecules), and/or bispecific/multispecific molecules may exhibit enhanced killing capability. The killing capability after combination of the above substances may be manifested as killing antigen expression-positive cells, optionally in the presence of effector cells, for example, via CDC, apoptosis, ADCC, phagocytosis, or via a combination of two or more of these mechanisms.

In the present invention, the term "directly or indirectly linked" refers to direct linkage via peptide bonds, or indirect linkage via a linker or via non-peptide linkage.

In the present invention, the term "PBMC" or "human peripheral blood mononuclear cells" generally refers to cells having a single nucleus in peripheral blood. For example, any blood cell having a round nucleus (i.e., lymphocytes, monocytes, or macrophages). These blood cells are key components of the immune system for resisting infection and adapting to invaders. The lymphocyte population is composed of CD4+ and CD8+ T cells, B cells and natural killer cells, CD14+ monocytes, and basophils/neutrophils/eosinophils/dendritic cells. Typically, these cells are separated from whole blood using FICOLL^{TM} (a hydrophilic polysaccharide that stratifies blood), wherein monocytes and lymphocytes form a buffy coat under the plasma layer. For example, "PBMC" refers to a cell population comprising at least T cells, and optionally comprising NK cells, NKT cells, and antigen-presenting cells.

In the present invention, the term "proliferation" refers to an increase in cell division (symmetric or asymmetric division of cells). "Proliferation" may refer to symmetric or asymmetric division of T cells. "Proliferation increase" occurs when the number of cells in a treated sample is increased as compared with the number of cells in an untreated sample.

In the present invention, the term "donor" includes any human or non-human animal. The term "non-human animal" includes all vertebrates, such as mammals and non-mammals, for example, non-human primates, sheep, dogs, cats, cattle, horses, chickens, amphibians, and reptiles, and may be a mammal, for example, a non-human primate, sheep, dog, cat, cattle, or horse.

In the present invention, the term "therapeutically effective amount" refers to an amount of the fusion protein of the present invention, or an amount of cells expressing the fusion protein of the present invention, sufficient to prevent or slow symptoms associated with a disease or condition (e.g., cancer). A therapeutically effective amount is related to the disease being treated, and one of ordinary skill in the art can readily determine the actual effective amount.

In the present invention, the term "drug" generally refers to a chemical compound or composition that, when properly administered to a patient, is capable of inducing a desired therapeutic effect.

In the present invention, the term "composition" denotes a mixture comprising one or more compounds described in the present invention, or physiologically/pharmaceutically acceptable salts or prodrugs thereof, with other chemical components, as well as other components such as physiologically/pharmaceutically acceptable carriers and excipients. The purpose of a pharmaceutical composition is to facilitate administration to an organism and to facilitate absorption of the active ingredient so as to exert biological activity. A therapeutic composition generally should be sterile and stable under conditions of manufacture and storage.

In the present invention, the term "vector" generally refers to a nucleic acid molecule capable of transporting another nucleic acid to which it is linked. One class of vectors is a "plasmid", which refers to a circular double-stranded DNA loop into which other DNA segments may be ligated. Another class of vectors is a viral vector, into which other DNA segments may be ligated into the viral genome. Certain vectors are capable of autonomous replication in the host cell into which they are introduced (e.g., bacterial vectors having a bacterial origin of replication and episomal mammalian vectors). Other vectors (e.g., non-episomal mammalian vectors) may be integrated into the genome of a host cell upon introduction into the host cell, thereby being replicated together with the host genome, such as naked RNA polynucleotides that cannot autonomously replicate, naked DNA polynucleotides, polynucleotides composed of DNA and RNA in the same strand, poly-lysine-coupled DNA or RNA, peptide-coupled DNA or RNA, liposome-coupled DNA, and the like. In addition, certain vectors are capable of directing expression of genes operably linked thereto. Such vectors are referred to in the present invention as "recombinant expression vectors" (or simply "expression vectors"). Generally, expression vectors used in recombinant DNA techniques are typically in the form of plasmids. In the present specification, "plasmid" and "vector" are used interchangeably, because plasmids are the most commonly used form of vector.

In the present invention, the term "adjuvant" generally refers to any substance that assists or modulates the action of a drug, including but not limited to an immunological adjuvant, which enhances or diversifies an immune response to an antigen.

In the present invention, the term "tumor" or "tumor cell" generally refers to or describes a physiological condition in mammals that is generally characterized by unregulated cell growth. Examples of tumors include, but are not limited to, carcinomas, lymphomas, blastomas (including medulloblastomas and retinoblastomas), sarcomas (including liposarcomas and synovial cell sarcomas), neuroendocrine tumors (including carcinoid tumors, gastrinomas, and islet cell carcinomas), mesotheliomas, schwannomas (including acoustic neuromas), meningiomas, adenocarcinomas, and melanomas. "Tumor" may further include "solid tumor", which refer to tumors selected from the group consisting of gastrointestinal cancer, pancreatic cancer, glioblastoma, cervical cancer, ovarian cancer, bladder cancer, hepatoma, breast cancer, colon cancer, rectal cancer, colorectal cancer, endometrial or uterine cancer, salivary gland cancer, renal cancer, prostate cancer, vulvar cancer, thyroid cancer, hepatic carcinoma, anal cancer, penile cancer, testicular cancer, esophageal cancer, bile duct tumors, and head and neck cancer.

In the present invention, the terms "about" and "approximately" generally refer to a statistically meaningful range of values. Such a range may be within an order of magnitude of a given value or range, and may include within 50%, preferably within 20%, more preferably within 10%, and most preferably within 5%. The permissible variation encompassed by the term "about" or "approximately" may depend on the particular system under study, and can be readily understood by one of ordinary skill in the art.

In the present invention, the terms "above", "below", "at most", and "at least" include the stated number itself.

In the present invention, the terms "include", "comprise", or "contain" are open-ended expressions, whereas "consist of" is a closed-ended expression; the former covers the latter, and the latter is a special form of the former.

### Embodiments of the Invention

The present invention provides improvements to therapies based on tumor-infiltrating lymphocytes ("TIL cells"). For example, the improvements of the present invention relate to targeting of the tumor microenvironment. For example, cells of the present invention that express a multispecific antigen-binding protein have one or more of the following characteristics: (1) increased cell proliferation capacity; (2) enhanced cytokine secretion capacity; (3) increased tumor cell killing capacity; (4) increased or stable exogenous gene transduction efficiency; (5) increased downstream signaling pathway strength; and/or (6) improved cell subpopulation proportions, for example, an increased proportion of activated cells, a reduced proportion of regulatory cells, a reduced proportion of exhausted cells, an increased proportion of central memory cells and/or naïve cells, a reduced proportion of apoptotic cells, and an increased proportion of stem cell-like cells.

In one aspect, an isolated cell is provided, which expresses a multispecific antigen-binding protein, wherein one antigen-binding fragment of the multispecific antigen-binding protein specifically binds to a surface antigen of the cell.

For example, the cell comprises a tumor-infiltrating lymphocyte (TIL). For example, the TIL is derived from tumor tissue, a tumor-associated lymph node with or without tumor metastasis, a tumor metastatic lesion, a fragment of paracancerous tissue, pleural effusion and/or ascites, and/or is derived from a cryopreserved TIL after resuscitation.

For example, the cell comprises a phagocyte, a lymphocyte, a neutrophil, an eosinophil, and/or a basophil. For example, the immune cell comprises a monocyte, a macrophage, and/or a dendritic cell. For example, the cell comprises a B cell, a T cell, a natural killer cell, and/or a natural killer-like T cell (NKT). For example, the cell comprises an αβ T cell and/or a γδ T cell. For example, the cell is an immune cell derived from stem cell differentiation, wherein the stem cell comprises an induced pluripotent stem cell (iPSC), an embryonic stem cell, a bone marrow stem cell, a cord blood stem cell, and/or a peripheral blood stem cell.

For example, the cell comprises an engineered immune receptor displayed on the cell surface, wherein the engineered immune receptor comprises a T cell receptor. For example, the engineered immune receptor specifically binds to an antigen expressed on a target cell. For example, the immune cell comprises a chimeric antigen receptor and/or a T cell receptor.

For example, the multispecific antigen-binding protein comprises a first antigen-binding fragment, wherein the first antigen-binding fragment binds CD3. For example, the first antigen-binding fragment comprises a first heavy chain variable region and a first light chain variable region, wherein the first heavy chain variable region comprises HCDR1 as set forth in SEQ ID NO: 11, HCDR2 as set forth in SEQ ID NO: 12, and HCDR3 as set forth in SEQ ID NO: 13, and wherein the first light chain variable region comprises LCDR1 as set forth in SEQ ID NO: 14, LCDR2 as set forth in SEQ ID NO: 15, and LCDR3 as set forth in SEQ ID NO: 16. For example, the first antigen-binding fragment comprises a first heavy chain variable region and a first light chain variable region, wherein the first heavy chain variable region comprises the sequence as set forth in SEQ ID NO: 17, and wherein the first light chain variable region comprises the sequence as set forth in SEQ ID NO: 18. For example, the first antigen-binding fragment comprises sequences of HCDR1-3 that are the same as those of the first heavy chain variable region as set forth in SEQ ID NO: 17, and comprises sequences of LCDR1-3 that are the same as those of the first light chain variable region as set forth in SEQ ID NO: 18. For example, the first antigen-binding fragment comprises the sequence as set forth in SEQ ID NO: 19. For example, the first antigen-binding fragment binds CD3. For example, the multispecific antigen-binding protein of the present invention comprises the first antigen-binding fragment as set forth in SEQ ID NO: 19 and a second antigen-binding fragment that binds a tumor-specific antigen.

For example, the multispecific antigen-binding protein comprises a second antigen-binding fragment, wherein the second antigen-binding fragment binds a tumor-specific antigen. For example, "tumor-associated antigen" (TAA) is well known in the art and refers to a molecule that is differentially expressed on and/or in cancer cells relative to non-cancerous cells of the same cell type. Non-limiting examples of TAAs include CD5, CD19, CD20, CD22, CD23, CD25, CD27, CD30, CD33, CD34, CD37, CD38, CD40, CD43, CD44v6, CD47, CD50, CD52, CD56, CD63, CD72a, CD74, CD78, CD79a, CD79b, CD86, CD134, CD137, CD138, CD248, CD319, αvβ3, α5β1, human epidermal growth factor receptor (EGFR or HER1), HER2, HER3, HER4, vascular endothelial growth factor receptor 1 (VEGFR-1), VEGFR-2, VEGFR-3, TRAIL-R2, carbohydrate antigen 19-9 (CA 19-9), carbohydrate antigen 125 (CA 125), carcinoembryonic antigen (CEA), mucin 1 (MUC 1), MUC2, MUC3, MUC4, MUC5, MUC7, ganglioside GD2, ganglioside GD3, ganglioside GM2, carbonic anhydrase IX (CAIX), SHH factor, melanoma chondroitin sulfate proteoglycan (MCSP), chondroitin sulfate proteoglycan 4 (CSPG4), six-transmembrane epithelial antigen of prostate (six-transmembrane epithelial antigen of prostate; STEAP), A33 antigen, desmoglein-2 (Dsg2), Dsg3, Dsg4, E-cadherin neoepitope, fetal nicotinic acetylcholine receptor (fnAChR), muellerian inhibitory substance receptor type II (MISIIR), tumor-associated antigen L6 (TAL6), Thomsen-Friedenreich (TF) antigen, EPHA1, EPHA2, EPHA3, EPHA4, EPHA7, EPHA8, EPHA10, EPHB4, cancer testis antigen (CTA), NY-BR1, tumor-associated glycoprotein 72 (TAG-72), α-fetoprotein (AFP), brother of the regulator of the imprinted site (BORIS), B cell activating factor (BAFF), extradomain-B fibronectin (EDB-FN), glycoprotein A33 (GPA33), tenascin-C (TNC), melanoma-associated antigen (MAGE), GAGE, BAGE, prostate stem cell antigen (PSCA), mesothelin, mucin-associated Tn, Sialyl Tn, globo H, stage-specific embryonic antigen-4 (SSEA-4), epithelial cell adhesion molecule (EpCAM), cytotoxic T lymphocyte-associated protein 4 (CTLA-4), programmed cell death 1 (PD-1), programmed cell death 1 ligand 1 (PD-L1), prostate-specific membrane antigen (PSMA), fibroblast activation protein (FAP), DLL3, BCMA, vascular cell adhesion protein 1 (VCAM-1), insulin-like growth factor receptor (IGFR), or hepatocyte growth factor receptor (HGFR).

For example, the multispecific antigen-binding protein comprises a second antigen-binding fragment, wherein the second antigen-binding fragment binds an antigen selected from the group consisting of epidermal growth factor receptor (EGFR), epidermal growth factor receptor variant III (EGFRvIII), fibroblast activation protein (FAP), DLL3, CD19, CD79b, CD37, prostate-specific membrane antigen (PSMA), prostate stem cell antigen (PSCA), interleukin-13 receptor α2 (IL-13Rα2), ephrin type-A receptor 1 (EphA1), human epidermal growth factor receptor 2 (HER2), mesothelin, cell surface-associated mucin 1 (MUC1), or cell surface-associated mucin 16 (MUC16).

For example, the second antigen-binding fragment of the present invention comprises an anti-TAA binding moiety, and in some examples, the anti-TAA binding moiety comprises a heavy chain variable region (VH) and a light chain variable region (VL). In some examples, the anti-TAA binding moiety is specific for CD20 (e.g., human CD20). In some examples, the anti-TAA binding moiety is specific for CD19 (e.g., human CD19). In some examples, the anti-TAA binding moiety is specific for EGFR (e.g., human EGFR). In some examples, the anti-TAA binding moiety is specific for HER2 (e.g., human HER2). In some examples, the anti-TAA binding moiety is specific for PSMA (e.g., human PSMA). In some examples, the anti-TAA binding moiety is specific for DLL3. In some examples, the anti-TAA binding moiety is specific for BCMA. In some examples, the anti-TAA binding moiety is specific for CEA (e.g., human CEA). In some examples, the anti-TAA binding moiety is specific for EpCAM (e.g., human EpCAM). In some examples, the anti-TAA binding moiety is specific for FAP (e.g., human FAP). In some examples, the anti-TAA binding moiety is specific for PD-L1 (e.g., human PD-L1). In some examples, the anti-TAA binding moiety is specific for CD38 (e.g., human CD38). In some examples, the anti-TAA binding moiety is specific for CD33 (e.g., human CD33). In some examples, the anti-TAA binding moiety is specific for HGFR (cMET) (e.g., human cMET). In some examples, the anti-TAA binding moiety is specific for CD47 (e.g., human CD47). In some examples, the anti-TAA binding moiety is specific for TRAIL-R2 (e.g., human TRAIL-R2). In some examples, the anti-TAA binding moiety is specific for mesothelin (e.g., human mesothelin). In some examples, the anti-TAA binding moiety is specific for GD2 (e.g., human GD2).

For example, the antigen-binding fragments described herein have suitable binding affinity to one or both of a target antigen (e.g., CD3 and TAA) or one or both epitopes thereof. As used herein, "binding affinity" refers to the apparent association constant, or KA. KA is the inverse of the dissociation constant (KD). The binding affinity (KD) to CD3 described herein is at least 100nM, 10nM, 1nM, 0.1nM, or lower (e.g., lower than 1nM or 0.1nM). Alternatively, the binding affinity (KD) to TAA described herein is at least 100nM, 10nM, 1nM, 0.1nM, or lower. Binding affinity (or binding specificity) is also determined by various methods, including equilibrium dialysis, equilibrium binding, gel filtration, ELISA, surface plasmon resonance, or spectroscopy (e.g., using fluorescence analysis).

For example, the second antigen-binding fragment described herein comprises a second heavy chain variable region and a second light chain variable region, wherein the second heavy chain variable region comprises HCDR1 as set forth in SEQ ID NO: 21, HCDR2 as set forth in SEQ ID NO: 22, and HCDR3 as set forth in SEQ ID NO: 23, and wherein the second light chain variable region comprises LCDR1 as set forth in SEQ ID NO: 24, LCDR2 as set forth in SEQ ID NO: 25, and LCDR3 as set forth in SEQ ID NO: 26. For example, the second antigen-binding fragment described herein comprises a second heavy chain variable region and a second light chain variable region, wherein the second heavy chain variable region comprises the sequence as set forth in SEQ ID NO: 27, and wherein the second light chain variable region comprises the sequence as set forth in SEQ ID NO: 28. For example, the second antigen-binding fragment described herein comprises sequences of HCDR1-3 that are identical to those of the second heavy chain variable region as set forth in SEQ ID NO: 27, and comprises sequences of LCDR1-3 that are identical to those of the second light chain variable region as set forth in SEQ ID NO: 28. For example, the second antigen-binding fragment described herein comprises the sequence as set forth in SEQ ID NO: 29. For example, the second antigen-binding fragment binds EGFR. For example, the multispecific antigen-binding protein of the present invention may comprise the second antigen-binding fragment as set forth in SEQ ID NO: 29 and a first antigen-binding fragment that binds an immune cell surface protein.

For example, the second antigen-binding fragment described herein comprises a second heavy chain variable region and a second light chain variable region, wherein the second heavy chain variable region comprises HCDR1 as set forth in SEQ ID NO: 31, HCDR2 as set forth in SEQ ID NO: 32, and HCDR3 as set forth in SEQ ID NO: 33, and wherein the second light chain variable region comprises LCDR1 as set forth in SEQ ID NO: 34, LCDR2 as set forth in SEQ ID NO: 35, and LCDR3 as set forth in SEQ ID NO: 36. For example, the second antigen-binding fragment described herein comprises a second heavy chain variable region and a second light chain variable region, wherein the second heavy chain variable region comprises the sequence as set forth in SEQ ID NO: 37, and wherein the second light chain variable region comprises the sequence as set forth in SEQ ID NO: 38. For example, the second antigen-binding fragment described herein comprises sequences of HCDR1-3 that are identical to those of the second heavy chain variable region as set forth in SEQ ID NO: 37, and comprises sequences of LCDR1-3 that are identical to those of the second light chain variable region as set forth in SEQ ID NO: 38. For example, the second antigen-binding fragment described herein comprises the sequence as set forth in SEQ ID NO: 39. For example, the second antigen-binding fragment binds EGFR. For example, the multispecific antigen-binding protein of the present invention may comprise the second antigen-binding fragment as set forth in SEQ ID NO: 39 and a first antigen-binding fragment that binds an immune cell surface protein.

For example, the second antigen-binding fragment described herein comprises a second heavy chain variable region and a second light chain variable region, wherein the second heavy chain variable region comprises HCDR1 as set forth in SEQ ID NO: 41, HCDR2 as set forth in SEQ ID NO: 42, and HCDR3 as set forth in SEQ ID NO: 43, and wherein the second light chain variable region comprises LCDR1 as set forth in SEQ ID NO: 44, LCDR2 as set forth in SEQ ID NO: 45, and LCDR3 as set forth in SEQ ID NO: 46. For example, the second antigen-binding fragment described herein comprises a second heavy chain variable region and a second light chain variable region, wherein the second heavy chain variable region comprises the sequence as set forth in SEQ ID NO: 47, and wherein the second light chain variable region comprises the sequence as set forth in SEQ ID NO: 48. For example, the second antigen-binding fragment described herein comprises sequences of HCDR1-3 that are identical to those of the second heavy chain variable region as set forth in SEQ ID NO: 47, and comprises sequences of LCDR1-3 that are identical to those of the second light chain variable region as set forth in SEQ ID NO: 48. For example, the second antigen-binding fragment described herein comprises the sequence as set forth in SEQ ID NO: 49. For example, the second antigen-binding fragment binds FAP. For example, the multispecific antigen-binding protein of the present invention may comprise the second antigen-binding fragment as set forth in SEQ ID NO: 49 and a first antigen-binding fragment that binds an immune cell surface protein.

For example, the second antigen-binding fragment described herein comprises a second heavy chain variable region and a second light chain variable region, wherein the second heavy chain variable region comprises HCDR1 as set forth in SEQ ID NO: 51, HCDR2 as set forth in SEQ ID NO: 52, and HCDR3 as set forth in SEQ ID NO: 53, and wherein the second light chain variable region comprises LCDR1 as set forth in SEQ ID NO: 54, LCDR2 as set forth in SEQ ID NO: 55, and LCDR3 as set forth in SEQ ID NO: 56. For example, the second antigen-binding fragment described herein comprises a second heavy chain variable region and a second light chain variable region, wherein the second heavy chain variable region comprises the sequence as set forth in SEQ ID NO: 57, and wherein the second light chain variable region comprises the sequence as set forth in SEQ ID NO: 58. For example, the second antigen-binding fragment described herein comprises sequences of HCDR1-3 that are identical to those of the second heavy chain variable region as set forth in SEQ ID NO: 57, and comprises sequences of LCDR1-3 that are identical to those of the second light chain variable region as set forth in SEQ ID NO: 58. For example, the second antigen-binding fragment described herein comprises the sequence as set forth in SEQ ID NO: 59. For example, the second antigen-binding fragment binds FAP. For example, the multispecific antigen-binding protein of the present invention may comprise the second antigen-binding fragment as set forth in SEQ ID NO: 59 and a first antigen-binding fragment that binds an immune cell surface protein.

For example, the antibody of the present invention may be delineated by a Kabat method, a Chothia method, an AbM method, a Contact method, or an IMGT method known in the art. For example, the method for delineating CDRs in the present invention is the Kabat method.

For example, the multispecific antigen-binding protein described herein comprises a first antigen-binding fragment and a second antigen-binding fragment, wherein the first antigen-binding fragment and the second antigen-binding fragment are linked via a peptide linker. The first antigen-binding fragment may be located at the N-terminus of the second antigen-binding fragment or at the C-terminus of the second antigen-binding fragment.

For example, the multispecific antigen-binding protein of the present invention comprises a BiTE. As used herein, a "bispecific T cell engager", "BiTE antibody construct", or "BiTE" means a polypeptide each comprising tandemly linked single-chain variable fragments (scFv) or nanobodies (VHH). Optionally, the scFv is linked via a linker (e.g., a glycine-rich linker). One scFv or VHH of the BiTE binds a T cell receptor (TCR) (e.g., binds a CD3ε subunit), and the other scFv or VHH binds a target antigen (e.g., a tumor-associated antigen).

For example, the multispecific antigen-binding protein of the present invention comprises a BiKE. As used herein, a "bispecific NK cell engager", "BiKE antibody construct", or "BiKE" means a polypeptide each comprising tandemly linked single-chain variable fragments (scFv) or nanobodies (VHH). Optionally, the scFv is linked via a linker (e.g., a glycine-rich linker). For example, one scFv or VHH of the BiKE binds CD16, and the other scFv or VHH binds a target antigen (e.g., a tumor-associated antigen).

For example, the multispecific antigen-binding protein of the present invention may comprise the second antigen-binding fragment as set forth in SEQ ID NO: 29 and a first antigen-binding fragment that binds CD3. For example, the multispecific antigen-binding protein of the present invention may comprise the second antigen-binding fragment as set forth in SEQ ID NO: 39 and a first antigen-binding fragment that binds CD3. For example, the multispecific antigen-binding protein of the present invention may comprise the second antigen-binding fragment as set forth in SEQ ID NO: 49 and a first antigen-binding fragment that binds CD3. For example, the multispecific antigen-binding protein of the present invention may comprise the second antigen-binding fragment as set forth in SEQ ID NO: 59 and a first antigen-binding fragment that binds CD3. For example, the present invention further provides a multispecific antigen-binding protein, wherein the multispecific antigen-binding protein comprises the sequence as set forth in SEQ ID NO: 30, 40, 50, and/or 60.

For example, the multispecific antigen-binding protein may comprise a signal peptide known in the art. For example, the multispecific antigen-binding protein may comprise the signal peptide as set forth in SEQ ID NO: 1 of the present invention. For example, the multispecific antigen-binding protein may comprise a tag polypeptide FLAG and/or 8His. For example, the multispecific antigen-binding protein may comprise the sequence as set forth in SEQ ID NO: 3 and/or 4 of the present invention. For example, the multispecific antigen-binding protein may be linked to a peptide segment comprising Thy1.1 or a fragment thereof. For example, the Thy1.1 comprises the amino acid sequence as set forth in SEQ ID NO: 6. For example, the Thy1.1 and the multispecific antigen-binding protein are linked via a cleavable peptide.

For example, the cleavable peptide may be used to link two or more components, such as molecules or peptides. For example, the cleavable peptide may be used to link two or more non-peptide molecules. For example, the cleavable peptide may be used to link two or more peptide molecules. For example, the cleavable peptide may be used to link different types of components, for example, wherein one or some components are non-peptide small-molecule substances and another or some components are peptides. For example, a substance linked via the cleavable peptide in the present invention may be referred to as a fusion protein or a fusion polypeptide (the two terms are used interchangeably in the present invention). For example, the cleavable peptide comprises T2A and/or P2A, and the cleavable peptide further optionally comprises a Furin peptide. For example, the Furin peptide is extended on the basis of T2A and/or P2A, and when T2A and/or P2A is cleaved, the Furin peptide may serve as a buffer to prevent an active ingredient peptide linked via T2A and/or P2A from being cleaved. For example, the amino acid sequence of the cleavable peptide is as set forth in SEQ ID NO: 5.

For example, the heavy chain variable region and the light chain variable region in the multispecific antigen-binding protein may be linked to each other via a linker (e.g., a glycine-based linker). For example, the heavy chain variable region and the light chain variable region in the multispecific antigen-binding protein may be linked via the sequence as set forth in SEQ ID NO: 2 of the present invention.

In another aspect, the present invention provides a method for producing a cell of the present invention, comprising causing the cell to artificially express the multispecific antigen-binding protein. For example, the cell is caused to artificially express a sequence as set forth in SEQ ID NO: 30, 40, 50, and/or 60. For example, the method of the present invention may transduce into the cell a nucleic acid sequence comprising a nucleic acid sequence encoding the sequence as set forth in SEQ ID NO: 7, 8, 9, and/or 10.

For example, the sequences mentioned in the present invention comprise sequences having at least about 80% homology to the sequences. For example, the sequences mentioned in the present invention comprise sequences having at least about 80%, at least about 85%, at least about 90%, at least about 95%, at least about 96%, at least about 97%, at least about 98%, or at least about 99% homology to the sequences.

In one aspect, the present invention provides a composition comprising a cell of the present invention and, optionally, a pharmaceutically acceptable carrier.

In one aspect, the present invention provides a kit, wherein the kit comprises a cell of the present invention and/or a composition of the present invention.

In one aspect, the present invention provides use of a cell of the present invention, a composition of the present invention, and/or a kit of the present invention in the preparation of a medicament, wherein the medicament is used for preventing, alleviating, and/or treating a disease. For example, the disease comprises a tumor.

In one aspect, a medicament for preventing and/or treating a disease and/or symptom is provided, comprising a cell of the present invention, a composition of the present invention, and/or a kit of the present invention. For example, the disease comprises a tumor.

In one aspect, a method for preventing and/or treating a disease and/or symptom is provided, comprising administering, to a subject in need thereof, a cell of the present invention, a composition of the present invention, and/or a kit of the present invention. For example, the disease comprises a tumor.

In one aspect, a cell of the present invention, a composition of the present invention, and/or a kit of the present invention is provided for use in preventing and/or treating a disease and/or symptom. For example, the disease comprises a tumor.

Without wishing to be bound by any theory, the examples below are merely used to illustrate the products, preparation methods, and uses of the present invention, and are not intended to limit the scope of the present invention.

### EXAMPLES

### Example 1. Method for culturing tumor-infiltrating lymphocyte (TIL) cells

### 1.1 Receipt and processing of tumor tissue

### 1.1.1 Tissue receipt

Tumor tissue and blood samples from a donor were received, sample information was checked and recorded, and corresponding sample labels were printed.

### 1.1.2 Tissue processing and culture

Sample tubes and blood collection tubes were disinfected with 75% alcohol and transferred into a biosafety cabinet. PBMCs in the blood samples were isolated and cryopreserved according to the above manual PBMC separation and cryopreservation operating procedure. A culture flask or a culture bag having a gas-permeable surface was provided, for example, a culture bag (Origen), and 300 mL of rewarmed complete medium was added. The complete medium could be optionally selected from X-vivo 15 medium or other commercially available T cell media, such as T cell media of brands including Stem Cell, Lonza, Thermo, Miltenyi, etc., and essential amino acids and antibiotics could be added, and IL-2 at a concentration of 300-9000 IU/mL (e.g., 1000-9000 IU/mL, e.g., 6000 IU/mL) was added. Several 10 cm culture dishes were provided, an appropriate amount of medium was added, tumor tissue was taken out from the sample tube into a 10 cm culture dish using sterile ophthalmic forceps, and the tissue was washed and the culture dish was replaced. Preliminary cutting was performed using ophthalmic scissors and ophthalmic forceps to remove adipose tissue and necrotic tissue, and each tissue block was further minced to a size of about 27 cubic millimeters. Non-suspended tumor tissue blocks were taken, an internal plunger was removed from a 20 mL syringe, the syringe was connected to the culture bag, and about 1 g of tissue blocks was transferred into the culture bag through the syringe using a pipette. The culture bag was placed into a CO₂ incubator for culture. The scissors and forceps were cleaned, preliminarily disinfected with 75% alcohol, ultrasonically cleaned, and then sterilized, thereby obtaining a first TIL population.

### 1.2 Step (A) in vitro expansion and harvest

### 1.2.1 Step (A) in vitro expansion

According to the cell growth status, medium was replenished or half of the medium was replaced every 3-7 days to ensure cell nutrition. Complete medium was used. The complete medium could be optionally selected from X-vivo 15 medium or other commercially available T cell media, such as T cell media of brands including Stem Cell, Lonza, Thermo, Miltenyi, etc., and essential amino acids and antibiotics could be added, and IL-2 at a concentration of 300-9000 IU/mL (e.g., 1000-9000 IU/mL, e.g., 6000 IU/mL) (Double-Crane and/or Sihuan) was added. After 3-14 days of step (A), for example, sampling and counting could be performed on day 13 or day 14, and when the cell number was between 5×10⁵ and 5×10⁸, the harvest step of step (A) was entered.

### 1.2.2 Harvest of step (A)

Cells at the end of the in vitro expansion of step (A) were collected and centrifuged, the medium was discarded, and the cells were washed once with PBS or normal saline, thereby obtaining TILs subjected to the in vitro expansion of step (A) (a second TIL population). Sampling and counting were performed, and about 5×10⁵ to 2×10⁸ cells were retained for a subsequent in vitro expansion step. About 5×10⁵ cells could be taken for quality control testing. The remaining cells were added with a cryopreservation solution and cryopreserved as cryopreserved preREP TIL in vitro cells.

### 1.3 Step (B) TIL activation

TILs subjected to the in vitro expansion of step (A) (the second TIL population) were continuously cultured, or the cryopreserved preREP TIL in vitro cells were resuscitated, to perform TIL activation of step (B).

Complete medium was used. The complete medium could be optionally selected from X-vivo 15 medium or other commercially available T cell media, such as T cell media of brands including Stem Cell, Lonza, Thermo, Miltenyi, etc., and essential amino acids and antibiotics could be added. The cell density was adjusted to 5×10⁵ to 2×10⁶ cells/mL, in a suspension 24-well plate at 1 mL/well, and IL-2 at a concentration of 300-9000 IU/mL (e.g., 1000-9000 IU/mL, e.g., 6000 IU/mL) was added. A T cell activator could also be added simultaneously to the medium of each TIL cell population, for example, a CD3 agonist and/or a CD28 agonist was added, for example, about 30 ng/mL CD3 antibody (Miltenyi Biotech, OKT3), about 30 ng/mL CD28 antibody (Merck, 15E8), magnetic beads were added at a ratio of magnetic beads to TIL of about 1:2-2:1 (Dynabeads with a diameter of about 1 to 10 µm, Thermo Fisher) and/or transACT was added at a ratio of transACT to TIL of about 1:100-1:2000 (with a diameter of about 100 to 500 nm, Miltenyi). Culture was performed for about 0-4 days to obtain a third TIL population.

### 1.4 TIL cell transduction

TILs activated in the above step (B) were transduced. One day before transduction, a 24-well suspension culture plate (24-well plate) was coated with a recombinant human fibronectin fragment (Retronectin, Takara) at a final concentration of 15 µg/mL. The plate was protected from light and stored at 4°C overnight for later use. The coated 24-well plate was taken out, the coating solution was aspirated and discarded, and a blocking solution containing 2% BSA was added for blocking at room temperature for 30 minutes. The blocking solution was aspirated and discarded, the plate was washed twice with a plate-washing solution containing 2.5% HEPES, and the plate-washing solution was aspirated and discarded.

Each experimental group was transduced with a retrovirus carrying a nucleic acid encoding the target polypeptide of the present invention (comprising the multispecific binding peptide segment EGFR-1-CD3 tandem scFv (BiTE1) shown in SEQ ID NO: 30; or comprising the multispecific binding peptide segment EGFR-2-CD3 tandem scFv (BiTE2) shown in SEQ ID NO: 40; or comprising the multispecific binding peptide segment FAP-1-CD3 tandem scFv (BiTE3) shown in SEQ ID NO: 50; or comprising the multispecific binding peptide segment FAP-2-CD3 tandem scFv (BiTE4) shown in SEQ ID NO: 60; for testing expression level only, a fusion protein comprising a multispecific binding peptide segment and a tag polypeptide as shown in any one of SEQ ID NO: 7-10 could be optionally expressed).

The plasmid construction method for the above retrovirus was as follows: a nucleic acid fragment encoding the target polypeptide of the present invention was synthesized, digested with EcoRI+NotI, and the exogenous fragment was recovered. Plasmid MP71 was digested with EcoRI+NotI, and the vector fragment was recovered. The exogenous fragment and the vector fragment were ligated with T4 Ligase to obtain a plasmid of a retrovirus that was capable of being used to express the target polypeptide of the present invention.

0.25-2 mL of retroviral solution was added to each well, followed by centrifugation at 32°C and 2000g for 2 hours; the blank control group was not subjected to cell transduction and was transduced using a control virus. The supernatant in the 24-well plate was discarded, and the TIL population activated in step 1.3 above was added to each well of the 24-well plate in a volume of 300-500 µL, with a cell concentration of about 1×10⁶ cells/mL. Centrifugation was performed at 30-32°C and 1000g for 10 minutes. After centrifugation, the culture plate was placed in a 37°C, 5% CO2 incubator and cultured for about 0-4 days to obtain a transduced TIL population, thereby obtaining a fourth TIL population.

### 1.5 Step (D) culture after TIL cell transduction

Feeder cells (irradiated healthy donor PBMC T cells) were added to the fourth TIL cell population for culture. The time for contact between TIL and feeder cells was required to be after a certain time Tₙ following contact between TIL and IL-2 and a T cell activator (e.g., a CD3 antibody or a nanomatrix comprising a CD3 antibody and a CD28 antibody, such as transACT) in step (B) (Tₙ for each experimental group could be 0 hours to 12 days, e.g., 24 hours or 48 hours). First, feeder cells mixed from 1-5 donors were resuscitated. Activated TIL cells and feeder cells were mixed at a ratio of TIL cells:feeder cells of about 1:200, transferred into a G-Rex100 culture flask or a gas-permeable bag, and supplemented with complete medium. Sampling and counting were performed every 1-3 days, and medium was replenished or half of the medium was replaced according to the cell status until the total cell number was greater than 1×10⁹ or step (D) in vitro expansion culture was performed for about 5 days to about 14 days, and the culture of step (D) in vitro expansion was terminated.

### 1.6 Harvest of tumor-infiltrating lymphocytes

Cells expanded in step (D) were taken and centrifuged, the culture medium supernatant was discarded, and the cells were washed three times with PBS or normal saline or a compound electrolyte solution, thereby obtaining TILs expanded in step (D) (a fifth TIL population). During the third wash, sampling and counting were performed, and according to the counting result, after the last centrifugation the supernatant was discarded, and 3×10⁶ cells were taken for quality control testing. All remaining cells were used as a final cell product, and a cryopreservation solution could be optionally added, and the cell density was adjusted to 1-3×10⁸ cells/mL for cryopreservation.

### Example 2. Preparation of TCR T cells

T cells from various sources such as peripheral blood and stem cell differentiation were taken, preferably PBMCs, and cultured. A nucleic acid encoding a TCR (e.g., a TCR targeting NY-ESO-1) was introduced into the above T cells according to methods known in the art, for example, by viral transduction, LNP transduction, electroporation, etc., thereby obtaining TCR-T cells. Before or after the TCR was introduced into the above T cells, each experimental group was transduced with a retrovirus carrying a nucleic acid encoding the target polypeptide of the present invention (e.g., BiTE1, BiTE2, BiTE3, or BiTE4), thereby obtaining TCR T cells transduced with the target polypeptide of the present invention. T cells not transduced with the target polypeptide were used as a blank control group (Control).

### Example 3. Detection of cell transduction efficiency

Flow cytometry was performed to detect cytokine expression in a TIL population obtained on day 7 or day 8 after gene editing with reference to Example 1 of the present application.

### Detection steps:

TILs of each experimental group were placed into a 96-well V-bottom plate and centrifuged, and an antibody mixed working solution was prepared for cell surface staining (BV421 anti-CD3, Biolegend 317344; APC anti-CD4, Biolegend 300514; FITC anti-CD8, Biolegend 344704; PE anti-Thy1.1 Invitrogen 12-0900-81), wherein the antibody concentration was 1:100, and the concentration of a cell viability detection dye (Invitrogen 65-0865-14) was 1:10000. After the cells were resuspended with 100µL/well of the antibody mixed working solution, incubation was performed at 2-8°C in the dark for 30 minutes. After staining was completed, an appropriate amount of PBS was added to wash the cells, centrifugation was performed at 600 g for 3 minutes, and the supernatant was discarded after centrifugation. After the cells were resuspended in PBS at 100µL/well, flow cytometric analysis was performed.

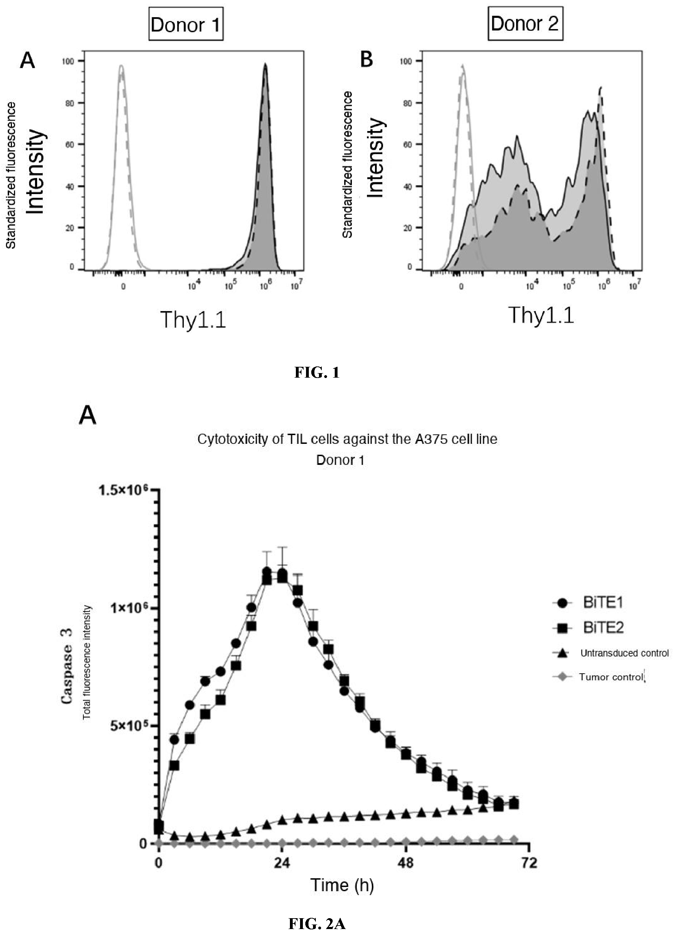

FIGs. 1A-1B showed transduction results of TIL cells derived from different donors. In FIG. 1A and FIG. 1B, a gray unfilled dashed line, a gray unfilled solid line, and a black filled solid line respectively represented a fluorescence minus one control (FMO), a blank control not transduced with the target polypeptide, TILs expressing BiTE1 (SEQ ID NO: 30), and TILs expressing BiTE2 (SEQ ID NO: 40). FIG. 1A showed TILs derived from cervical tumors, and FIG. 1B showed TILs derived from melanoma tumors.

By detecting the staining results of the Thy1.1 marker, the results showed that the Thy1.1 markers of TIL cells derived from two donors without gene editing were negative, and the Thy1.1 markers of TIL cells derived from two donors after gene editing were partially positive. Since the target polypeptide of the present invention and the Thy1.1 marker were encoded by the same plasmid and were located upstream of the Thy1.1 marker, the target polypeptide of the present invention was successfully expressed in TIL cells.

### Example 4. Detection of cell killing capability

TIL populations obtained on day 7 or day 8 after gene editing were subjected to detection of killing capability against tumor cells.

### Detection steps:

Tumor target cells were seeded in advance into a 96-well flat-bottom plate one day earlier. On the next day, TIL cells of each group were co-cultured with the target cells at an effector-to-target ratio of 1:1, with 100 µL each of target cells and TIL cells. Three replicate wells were set for each group, and a control group containing only target cells was also set.

According to the instructions of the apoptosis detection reagent (SuperViewTM 488 Caspase-3 Assay Kit, UEL S6007L), the apoptosis detection reagent was added at 1.5 µL/well and was diluted by adding culture medium at 18.5 µL/well. An Incucyte recorder (Sartorius) was used to record Caspase 3 activity to analyze the killing capability of TIL cells against target cells, and recording was performed once every 3 hours.

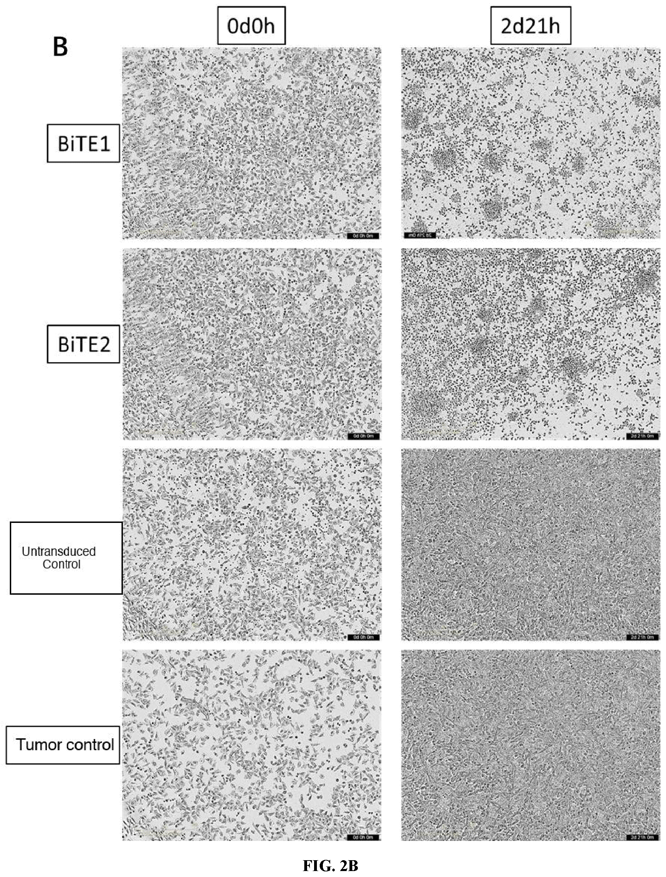

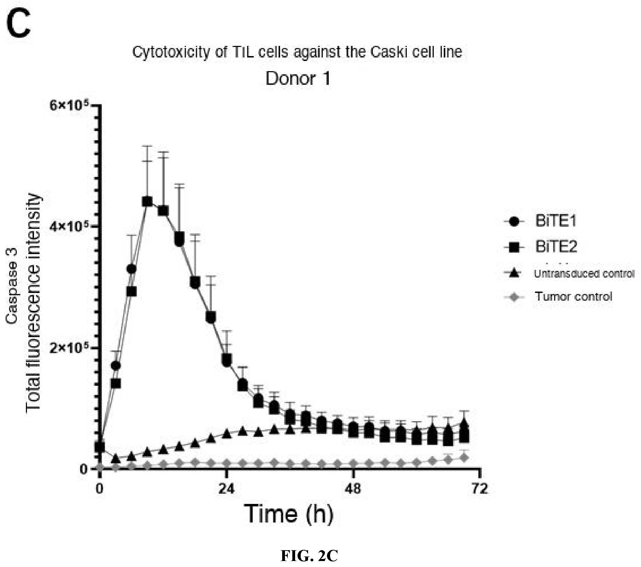

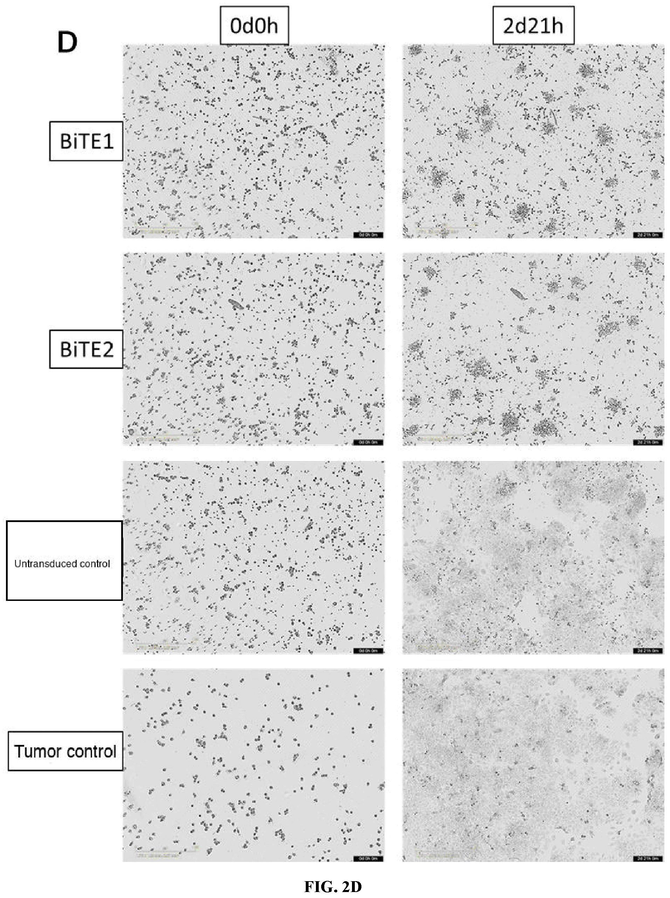

FIGs. 2A-2D show the detection results of the killing capability of TIL cells transduced with the target polypeptide of the present invention. The significance tests in the figures were all comparisons with untransduced TIL. FIG. 2A shows a killing curve, generated according to Caspase 3 activity, in a killing test of TIL cells against A375 cells. FIG. 2B shows the cell morphology of each group at each experimental time point scanned by Incucyte S3 in the killing test of TIL cells against A375. FIG. 2C shows a killing curve, generated according to Caspase 3 activity, in a killing test of TIL cells against Caski cells. FIG. 2D shows the cell morphology of each group at each experimental time point scanned by Incucyte S3 in the killing test of TIL cells against Caski. FIGs. 2A to 2D were cervical tumor-derived TIL.

The results showed that, compared with the untransduced control group, the gene-edited TIL cells had a more significant target-cell killing capability against A375 or Caski cells, and the killing speed was faster; after about 24 hours of co-incubation, the target cells could be substantially completely cleared. In the Incucyte scan images, it was observed that after 2 days and 21 hours of co-incubation, there were almost no tumor target cells in the gene-edited TIL cell group, while tumor target cells were massively expanded in the untransduced control group and the control group containing only target cells.

### Example 5. Detection of cytokine secretion of TIL cells

At 24 hours after the start of co-incubation in the killing capability detection of Example 4 above, supernatants of each group were collected for detection of cytokine secretion.

### Detection steps:

The cytokine secretion detection method could refer to the instructions of the cytokine detection kit (BD 560484). Specifically, the lyophilized powder of the human Th1/Th2/Th17 cytokine standard (BD) was reconstituted with 2 mL Assay Diluent diluent (BD) (the concentration of each cytokine in the standard stock solution was 5000 pg/mL) and was subjected to gradient dilution in sequence: 1:2, 1:4, 1:8, 1:16, 1:32, 1:64, 1:128, 1:256, 1:512, 1:1024, and was labeled as "standard tubes". One tube containing only Assay Diluent (assay diluent) was used as a reference. Each Capture Beads (BD) was added at 2 µL/Beads/well, and then PE Detection Reagent (BD) was added at 10 µL/well and mixed to prepare a mixture, which was added into a V-bottom 96-well plate at 24 µL/well. Subsequently, each standard and the supernatant of the experimental group were added at 10 µL/well and mixed, and incubation was performed at room temperature in the dark for 3 hours. After incubation, 200 µL Wash Buffer (BD) was added to each well, followed by centrifugation at 500 g for 3 minutes. After centrifugation, 100 µL Wash Buffer (BD) was added to each well for resuspension, and flow cytometric analysis was performed.

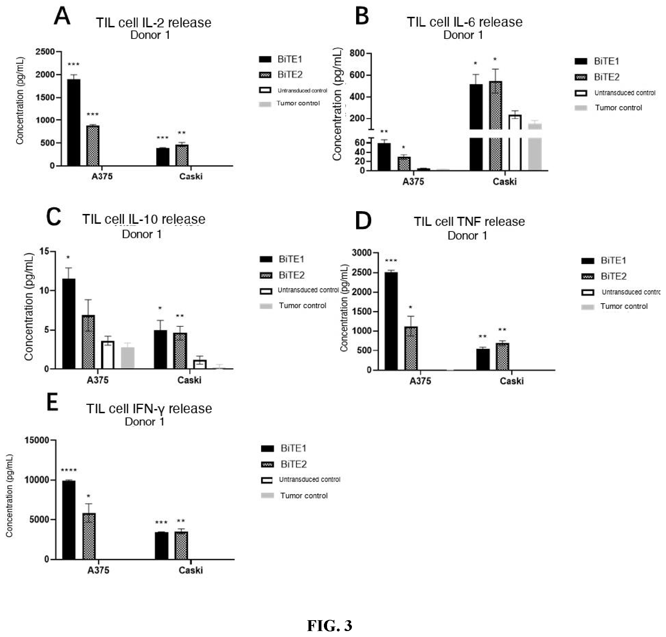

FIGs. 3A-3E show the concentrations of various cytokines in the supernatants after 24 hours of co-incubation of TIL cells of each group with A375 or Caski cells. The significance tests in the figures were all comparisons with the untransduced control group. FIGs. 3A to 3E were cervical tumor-derived TIL.

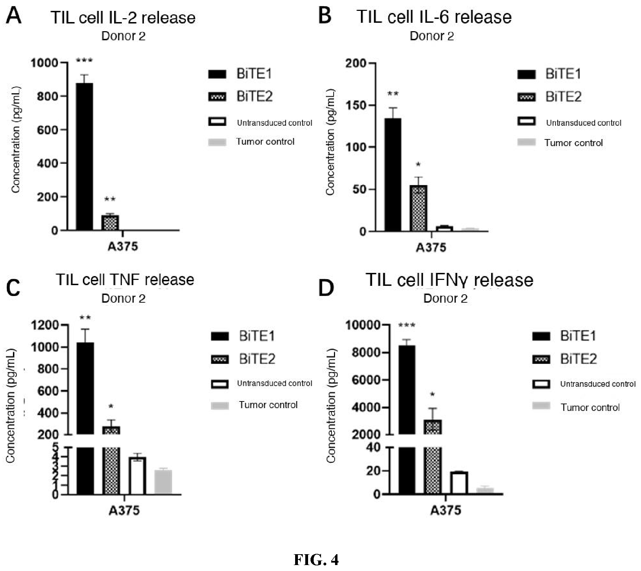

FIGs. 4A-4D show the concentrations of various cytokines in the supernatants after 24 hours of co-incubation of TIL cells of each group with A375 cells. The significance tests in the figures were all comparisons with the untransduced control group. FIGs. 4A to 4D were melanoma-derived TIL.

The results showed that, after co-incubation with target cells, the gene-edited TIL cells had higher secretion capabilities of IL-2, IL-6, IL-10, TNF and IFN-γ.

### Example 6. Detection of promotion of proliferation capability of TIL

The transduced TIL populations obtained in step 1.5 of Example 1 above were seeded into a 96-well plate after transduction and expansion. T cells not transduced with virus were used as a blank control group. For the stimulation group, the stimulation condition was TransACT (diameter about 100 to 500 nm, Miltenyi); the unstimulated group was denoted as medium, and the stimulated group was labeled as transact. After 2-3 days of stimulation, each group was analyzed for cell expansion using a CTG kit, and the CTG kit detection method was as in Example 4.

The results showed that, after expression of the target polypeptide of the present invention, the cells could have better proliferation capability.

### Example 7. Detection of proliferation capability of TCRT cells

An anti-CD3 antibody (e.g., OKT3) was coated on a 96-well plate (those subjected to antibody coating were denoted as CD3 coating, and those not subjected to antibody coating were denoted as medium), and incubation was performed at 37°C for 2 hours. Then, PBMC-TCR T cells transduced with the target polypeptide of the present invention obtained in the above examples were seeded into a 96-well plate after transduction and expansion. After 3 days, each group was analyzed for cell expansion using a CTG kit. According to the instructions of the CTG kit (CellTiter-Glo Luminescent Cell Viability Assay, Promega), a CTG reaction solution was prepared by mixing a CTG substrate (CellTiter-Glo Substrate) with a CTG buffer (CellTiter-Glo Buffer). The cell suspension to be tested was added into a 96-well microplate at 50 µL/well, and wells containing only culture medium were set as background values of fluorescence. An equal volume of the CTG reaction solution was added to each well, shaking was performed on a horizontal shaker for 2 minutes, and the plate was allowed to stand at room temperature for 10 minutes to stabilize the fluorescence signal, and then fluorescence values were read.

The results showed that, after expression of the target polypeptide of the present invention, the cells could have better proliferation capability.

### Example 8. Detection of cytokine secretion capability of TCRT cells

Anti-CD3 was coated on a 96-well plate, and incubation was performed at 37°C for 2 hours. Then, PBMC-TCR T cells transduced with the target polypeptide of the present invention obtained in the above examples were seeded into a 96-well plate after transduction and expansion. After 24-48 hours, supernatants were collected, and a Cytometric Bead Array (CBA) kit (BD) was used to analyze secretion of cytokines IL-2, TNF and IFN-γ.

The specific method referred to the instructions of the cytokine detection kit. Specifically, the lyophilized powder of the human Th1/Th2/Th17 cytokine standard was reconstituted with 2 mL Assay Diluent diluent (the concentration of each cytokine in the standard stock solution was 5000 pg/mL) and was subjected to gradient dilution in sequence: 1:2, 1:4, 1:8, 1:16, 1:32, 1:64, 1:128, 1:256, 1:512, 1:1024, and was labeled as "standard tubes". One tube containing only Assay Diluent diluent was used as a reference. Each Capture Beads (BD) was added at 2 µL/Beads/well, and then PE Detection Reagent detection reagent was added at 10 µL/well and mixed to prepare a mixture (mix), which was added into a V-bottom 96-well plate at 22 µL/well. Subsequently, each standard and the supernatant of the experimental group were added at 10 µL/well and mixed, and incubation was performed at room temperature in the dark for 3 hours. After incubation, 200 µL Wash Buffer (BD) was added to each well, followed by centrifugation at 500 g for 3 minutes. After centrifugation, 100 µL Wash Buffer (BD) was added to each well for resuspension, and flow cytometric analysis was performed.

The results showed that, after expression of the target polypeptide of the present invention, the cells could have enhanced cytokine secretion capability.

### Example 9. Detection of tumor cell killing capability of TCRT cells

GFP-labeled A375 tumor cells (A375-GFP) were seeded into a 96-well plate. After the cells adhered, PBMC-TCR T cells transduced with the target polypeptide of the present invention obtained in the above examples were added to each well for co-culture. According to the instructions of the apoptosis detection reagent (Incucyte Caspase-3/7 Green Dye for Apoptosis, Sartorius), Incucyte^{®} Caspase-3/7 Green Dye for Apoptosis was added at 0.2 µL/well, and Caspase 3/7 Green Dye was diluted by adding culture medium at 25 µL/well. An Incucyte recorder (Sartorius) was used to record Caspase 3/7 activity to analyze the killing capability against tumor cells, and recording was performed once every 3 hours, with a total recording duration of about 3 days.

The results showed that, after expression of the target polypeptide of the present invention, the cells could have enhanced tumor cell killing capability.

### Example 10. In vivo efficacy detection

Under sterile conditions, a tumor cell line was resuscitated and stably expanded in vitro for 3-5 passages, the cells were grown to the logarithmic growth phase, the cells were harvested and counted, the cell density was adjusted, and subcutaneous inoculation into NOG mice was performed in an SPF-grade animal facility. Tumor volume was measured regularly until the tumor volume had grown, and then the mice were randomly grouped.

The NT group was used as a negative control, and the mice were intravenously injected with TIL cells without gene editing; the mice in each experimental group were intravenously injected with the gene-edited TIL cells of the present invention, and IL-2 was administered by intraperitoneal injection as adjuvant therapy. The results showed that, after expression of the target polypeptide of the present invention, the cells could have the capability of inhibiting tumor growth.

The foregoing detailed description was provided by way of explanation and examples, and was not intended to limit the scope of the appended claims. Various modifications of the embodiments exemplified in the present invention were apparent to those of ordinary skill in the art, and were intended to be within the scope of the appended claims and equivalents thereof.

## Claims

1. An isolated cell, comprising expression of a multispecific antigen-binding protein, wherein one antigen-binding fragment of the multispecific antigen-binding protein specifically binds to a surface antigen of the cell.

2. The cell according to claim 1, wherein the cell comprises tumor-infiltrating lymphocytes (TIL).

3. The cell according to claim 2, wherein the TIL is derived from tumor tissue, tumor-associated lymph nodes with or without tumor metastasis, tumor metastatic lesions, fragments of paracancerous tissue, pleural effusion and/or ascites, and/or is derived from TIL that is resuscitated after cryopreservation.

4. The cell according to any one of claims 1-3, wherein the cell comprises phagocytes, lymphocytes, neutrophils, eosinophils, and/or basophils.

5. The cell according to any one of claims 1-4, wherein the cell comprises B cells, T cells, natural killer cells, and/or natural killer-like T cells (NKT).

6. The cell according to any one of claims 1-5, wherein the cell comprises αβ T cells and/or γδ T cells.

7. The cell according to any one of claims 1-6, wherein the cell is derived from immune cells differentiated from stem cells, wherein the stem cells comprise induced pluripotent stem cells (iPSC), embryonic stem cells, bone marrow stem cells, cord blood stem cells, and/or peripheral blood stem cells.

8. The cell according to any one of claims 1-7, wherein the cell comprises an engineered immune receptor displayed on the cell surface, and the engineered immune receptor comprises a T cell receptor.

9. The cell according to any one of claims 1-8, wherein the multispecific antigen-binding protein comprises a first antigen-binding fragment, and the first antigen-binding fragment binds CD3.

10. The cell according to claim 9, wherein the first antigen-binding fragment comprises a first heavy chain variable region and a first light chain variable region, the first heavy chain variable region comprises HCDR1 as shown in SEQ ID NO: 11, HCDR2 as shown in SEQ ID NO: 12, and HCDR3 as shown in SEQ ID NO: 13, and the first light chain variable region comprises LCDR1 as shown in SEQ ID NO: 14, LCDR2 as shown in SEQ ID NO: 15, and LCDR3 as shown in SEQ ID NO: 16.

11. The cell according to any one of claims 9-10, wherein the first antigen-binding fragment comprises a first heavy chain variable region and a first light chain variable region, the first heavy chain variable region comprises the sequence as shown in SEQ ID NO: 17 or a sequence having at least about 80% homology to the sequence, and the first light chain variable region comprises the sequence as shown in SEQ ID NO: 18 or a sequence having at least about 80% homology to the sequence.

12. The cell according to any one of claims 9-11, wherein the first antigen-binding fragment comprises the sequence as shown in SEQ ID NO: 19 or a sequence having at least about 80% homology to the sequence.

13. The cell according to any one of claims 1-12, wherein the multispecific antigen-binding protein comprises a second antigen-binding fragment, and the second antigen-binding fragment binds a tumor-specific antigen.

14. The cell according to claim 13, wherein the second antigen-binding fragment binds an antigen selected from the group consisting of: DLL3, epidermal growth factor receptor (EGFR), epidermal growth factor receptor variant III (EGFRvIII), fibroblast activation protein (FAP), CD19, CD79b, CD37, prostate-specific membrane antigen (PSMA), prostate stem cell antigen (PSCA), interleukin-13 receptor α2 (IL-13Rα2), ephrin type-A receptor 1 (EphA1), human epidermal growth factor receptor 2 (HER2), mesothelin, mucin 1 cell surface associated (MUC1), or mucin 16 cell surface associated (MUC16).

15. The cell according to any one of claims 13-14, wherein the second antigen-binding fragment comprises a second heavy chain variable region and a second light chain variable region, the second heavy chain variable region comprises HCDR1 as shown in SEQ ID NO: 21, HCDR2 as shown in SEQ ID NO: 22, and HCDR3 as shown in SEQ ID NO: 23, and the second light chain variable region comprises LCDR1 as shown in SEQ ID NO: 24, LCDR2 as shown in SEQ ID NO: 25, and LCDR3 as shown in SEQ ID NO: 26.

16. The cell according to any one of claims 13-15, wherein the second antigen-binding fragment comprises a second heavy chain variable region and a second light chain variable region, the second heavy chain variable region comprises the sequence as shown in SEQ ID NO: 27 or a sequence having at least about 80% homology to the sequence, and the second light chain variable region comprises the sequence as shown in SEQ ID NO: 28 or a sequence having at least about 80% homology to the sequence.

17. The cell according to any one of claims 13-16, wherein the second antigen-binding fragment comprises the sequence as shown in SEQ ID NO: 29 or a sequence having at least about 80% homology to the sequence.

18. The cell according to any one of claims 13-14, wherein the second antigen-binding fragment comprises a second heavy chain variable region and a second light chain variable region, the second heavy chain variable region comprises HCDR1 as shown in SEQ ID NO: 31, HCDR2 as shown in SEQ ID NO: 32, and HCDR3 as shown in SEQ ID NO: 33, and the second light chain variable region comprises LCDR1 as shown in SEQ ID NO: 34, LCDR2 as shown in SEQ ID NO: 35, and LCDR3 as shown in SEQ ID NO: 36.

19. The cell according to any one of claims 13-14 and 18, wherein the second antigen-binding fragment comprises a second heavy chain variable region and a second light chain variable region, the second heavy chain variable region comprises the sequence as shown in SEQ ID NO: 37 or a sequence having at least about 80% homology to the sequence, and the second light chain variable region comprises the sequence as shown in SEQ ID NO: 38 or a sequence having at least about 80% homology to the sequence.

20. The cell according to any one of claims 13-14 and 18-19, wherein the second antigen-binding fragment comprises the sequence as shown in SEQ ID NO: 39 or a sequence having at least about 80% homology to the sequence.

21. The cell according to any one of claims 13-14, wherein the second antigen-binding fragment comprises a second heavy chain variable region and a second light chain variable region, the second heavy chain variable region comprises HCDR1 as shown in SEQ ID NO: 41, HCDR2 as shown in SEQ ID NO: 42, and HCDR3 as shown in SEQ ID NO: 43, and the second light chain variable region comprises LCDR1 as shown in SEQ ID NO: 44, LCDR2 as shown in SEQ ID NO: 45, and LCDR3 as shown in SEQ ID NO: 46.

22. The cell according to any one of claims 13-14 and 21, wherein the second antigen-binding fragment comprises a second heavy chain variable region and a second light chain variable region, the second heavy chain variable region comprises the sequence as shown in SEQ ID NO: 47 or a sequence having at least about 80% homology to the sequence, and the second light chain variable region comprises the sequence as shown in SEQ ID NO: 48 or a sequence having at least about 80% homology to the sequence.

23. The cell according to any one of claims 13-14 and 21-22, wherein the second antigen-binding fragment comprises the sequence as shown in SEQ ID NO: 49 or a sequence having at least about 80% homology to the sequence.

24. The cell according to any one of claims 13-14, wherein the second antigen-binding fragment comprises a second heavy chain variable region and a second light chain variable region, the second heavy chain variable region comprises HCDR1 as shown in SEQ ID NO: 51, HCDR2 as shown in SEQ ID NO: 52, and HCDR3 as shown in SEQ ID NO: 53, and the second light chain variable region comprises LCDR1 as shown in SEQ ID NO: 54, LCDR2 as shown in SEQ ID NO: 55, and LCDR3 as shown in SEQ ID NO: 56.

25. The cell according to any one of claims 13-14 and 24, wherein the second antigen-binding fragment comprises a second heavy chain variable region and a second light chain variable region, the second heavy chain variable region comprises the sequence as shown in SEQ ID NO: 57 or a sequence having at least about 80% homology to the sequence, and the second light chain variable region comprises the sequence as shown in SEQ ID NO: 58 or a sequence having at least about 80% homology to the sequence.

26. The cell according to any one of claims 13-14 and 24-25, wherein the second antigen-binding fragment comprises the sequence as shown in SEQ ID NO: 59 or a sequence having at least about 80% homology to the sequence.

27. The cell according to any one of claims 1-26, wherein the multispecific antigen-binding protein comprises a first antigen-binding fragment and a second antigen-binding fragment, and the first antigen-binding fragment and the second antigen-binding fragment are linked by a peptide linker.

28. The cell according to any one of claims 1-27, wherein the multispecific antigen-binding protein comprises the sequence as shown in SEQ ID NO: 30, 40, 50, and/or 60 or a sequence having at least about 80% homology to the sequence.

29. A method for producing the cell according to any one of claims 1-28, comprising causing the cell to artificially express the multispecific antigen-binding protein.

30. A composition, comprising the cell according to any one of claims 1-28, and optionally a pharmaceutically acceptable carrier.

31. A kit, wherein the kit comprises the cell according to any one of claims 1-28 and/or the composition according to claim 30.

32. Use of the cell according to any one of claims 1-28, the composition according to claim 30, and/or the kit according to claim 31 in the manufacture of a medicament, wherein the medicament is used for preventing, alleviating, and/or treating a disease (preferably a tumor).

33. A medicament for preventing and/or treating a disease and/or symptom (preferably a tumor), comprising the cell according to any one of claims 1-28, the composition according to claim 30, and/or the kit according to claim 31.

34. A method for preventing and/or treating a disease and/or symptom (preferably a tumor), comprising administering, to a subject in need thereof, the cell according to any one of claims 1-28, the composition according to claim 30, and/or the kit according to claim 31.

35. Use of the cell according to any one of claims 1-28, the composition according to claim 30, and/or the kit according to claim 31 for preventing and/or treating a disease and/or symptom (preferably a tumor).
